# EUROPEAN PATENT SPECIFICATION

(11) **EP 3 409 116 B1**
(45) Date of publication and mention of the grant of the patent: **09.08.2023**
(21) Application number: 16888214.0
(22) Date of filing: 28.12.2016
(51) Int. Cl.: A01N 47/36, A01G 7/00, A01H 3/00, A01P 21/00, C12N 15/82

(54) **USE OF ALLANTOIN FOR ENHANCING HIGH-TEMPERATURE RESISTANCE IN PLANT**
VERWENDUNG VON ALLANTOIN ZUR VERBESSERUNG DER HOCHTEMPERATURBESTÄNDIGKEIT IN PFLANZEN
UTILISATION DE L'ALLANTOÏNE POUR AMÉLIORER LA RÉSISTANCE AUX TEMPÉRATURES ÉLEVÉES DES PLANTES

(30) Priority: 29.01.2016 JP 2016016383; 07.03.2016 JP 2016043503
(43) Date of publication of application: 05.12.2018
(73) Proprietor: Kaneka Corporation, Osaka-shi, Osaka 530-8288 (JP)
(72) Inventor: SAKAMOTO Atsushi, Higashihiroshima-shi Hiroshima 739-8526 (JP); SHIMADA Hiroshi, Higashihiroshima-shi Hiroshima 739-8526 (JP); TANAKA Shoma, Higashihiroshima-shi Hiroshima 739-8526 (JP); NOJIRI Masutoshi, Takasago-shi Hyogo 676-8688 (JP); FU Yu, Takasago-shi Hyogo 676-8688 (JP); KIZAKI Noriyuki, Takasago-shi Hyogo 676-8688 (JP); KITANO Mitsuaki, Takasago-shi Hyogo 676-8688 (JP)
(74) Representative: Hoffmann Eitle
(86) International application number: PCT/JP2016/089061
(87) International publication number: WO 2017/130630

(56) References cited:
- WO-A1-2005/094580
- WO-A1-2009/095922
- J. LARKINDALE ET AL: "Protection against Heat Stress-Induced Oxidative Damage in Arabidopsis Involves Calcium, Abscisic Acid, Ethylene, and Salicylic Acid", PLANT PHYSIOLOGY, vol. 128, no. 2, 1 February 2002 (2002-02-01), pages 682-695, XP055589152, Rockville, Md, USA ISSN: 0032-0889, DOI: 10.1104/pp.010320
- WATANABE, S. ET AL.: "The purine metabolite allantoin enhances abiotic stress tolerance through synergistic activation of abscisic acid metabolism", PLANT CELL ENVIRON., vol. 37, 2014, pages 1022-1036, XP002791351,
- SHUNSUKE WATANABE et al.: "The purine metabolite allantoin enhances abiotic stress tolerance through synergistic activation of abscisic acid metabolism.", Plant, Cell and Environment, vol. 37, 2014, pages 1022-1036, XP055531804,
- DIRK V. CHARLSON et al.: "Allantoate amidohydrolase transcript expression is independent of drought tolerance in soybean", Journal of Experimental Botany, vol. 60, no. 3, 2009, pages 847-851, XP055531806,
- SHOMA TANAKA et al.: "Impact of allantoin accumulation on heat shock tolerance in Arabidopsis", Abstract Book Annual Meeting of JSPP 2016, vol. 57th, 11 March 2016 (2016-03-11), page 317, XP009512795,
- HIROSHI TAKAGI et al.: "Allantoin, a stress- related purine metabolite, can activate jasmonate signaling in a MYC2-regulated and abscisic acid-dependent manner", Journal of Experimental Botany, vol. 67, no. 8, 1 March 2016 (2016-03-01), pages 2519-2532, XP055531811,

## Description

### Technical Field

The present invention relates to a high temperature stress resistance-improving agent, a method for improving high temperature stress resistance, a whitening suppressor, and a DREB2A gene expression promoter of a plant.

### Background Art

Plants remain alive while they are exposed to a wide variety of environmental stress, such as high temperature and dehydration. Unlike animals, it is difficult for plants to migrate by themselves. Accordingly, the mechanism for protecting themselves from environmental stress has developed in plants.

The dehydration stress responsive element (DRE) is a sequence, the presence of which has been verified as a result of promoter analysis of RD29A as a water stress inducible gene in the genome of *Arabidopsis thaliana.* The DRE binding protein (DREB) is a transcription factor isolated as a protein that binds to DRE. Among DREBs, DREB2A is an APETALA2/ethylene responsive element-binding factor-type (AP2/ERF-type) transcription factor, which was isolated as a DRE-recognizing protein (Non-Patent Document 3). Since DREB2A is strongly induced by dehydration stress and high salt concentration stress, DREB2A is considered to be a transcription factor that functions under dehydration stress and high temperature stress conditions (Non-Patent Document 1). DREB2A activity is regulated after translation, and a region of 30 amino acids adjacent to the AP2/ERF DNA binding domain is considered to play a key role in post-translational protein regulation. DREB2A CA derived from DREB2A via deletion of such region has activity, and DREB2A CA- overexpressing *Arabidopsis thaliana* exhibits a dwarf phenotype. Thus, DREB2A CA has been verified to improve dehydration stress resistance to a significant extent (Non-Patent Document 2).

Non-Patent Document 3 and Patent Document 1 each report the mechanism of a plant in which DREB2A induces a target gene when the plant receives high temperature stress. Non-Patent Document 3 and Patent Document 1 each propose the mechanism, such that a protein DPB3-1(or NF-YC10) interacts with DREB2A and induction of high temperature stress resistance gene expression is promoted by DREB2A. Also, Non-Patent Document 3 demonstrates that the DPB3-1/DREB2A interaction would not affect the expression of dehydration stress-inducible genes.

Patent Document 2 describes that a rooting rate is improved and the life of cut flower is prolonged in a transgenic plant into which the DREB2A gene had been introduced.

Meanwhile, allantoin (5-ureidohydantoin) is an intermediate product that is generated during the process of degradation of nucleic acid bases (purine bases). In plant bodies, allantoin is generated from 5-hydroxyisouric acid with the aid of allantoin synthase (AS) and then degraded into allantoic acid with the aid of allantoinase (ALN).

Non-Patent Document 4 reports that an aln-1 mutant derived from *Arabidopsis thaliana* by deletion of the ALN gene to accumulate allantoin in the plant body has higher dehydration stress resistance than wild-type plants.

Non-Patent Document 5 reports that plant growth is promoted via application of allantoin to *Arabidopsis thaliana.*

Non-Patent Document 6 describes the application of abscisic acid (ABA) to *Aradopsis* seedlings subjected to heat stress.

Patent Document 3 discloses a method of protecting a plant from stress via application of ureide, such as allantoin, to a plant. Patent Document 3 also discloses that a plant is damaged by oxidative stress upon environmental disturbance, such as droughts or coldness and that a plant is protected from damage since the scavenger pathway is promoted by high ureide concentration.

### Citation List

### Patent Documents

Patent Document 1: WO 2013/111755
Patent Document 2: JP Patent No. 4219711
Patent Document 3: US 2010/0333237

### Non-Patent Documents

Non-Patent Document 1: Sakuma, Y. et al., Proc. Natl. Acad. Sci., U.S.A., 103: 18822-18827, 2006
Non-Patent Document 2: Sakuma, Y. et al., Plant Cell 18: 1292-1309, 2006
Non-Patent Document 3: Sato, H., Plant Cell 26: 4954-4973, 2014
Non-Patent Document 4: Watanabe, S. et al., Plant Cell Environ., 37: 1022-1036, 2014
Non-Patent Document 5: Watanabe et al., Abstracts of the 55th Annual Meeting of the Japanese Society of Plant Physiologists, PF044 (0461), 2014
Non-Patent Document 6: Larkindale et al., Plant Physiology 123(2) : 682-695, 2002

### Summary of Invention

### Technical Problem

When plants receive high temperature stress, problems such as whitening, withering, or leaf shrinkage of plants arise, and plants cannot occasionally survive if high temperature stress is excessive. Thus, an improvement in high temperature stress resistance of plants, such as crops, has been desired.

As described above, allantoin is known to improve stress resistance, such as dehydration stress resistance or low temperature stress resistance, in plants. In the past, however, the correlation between high temperature stress resistance and allantoin has not yet been investigated.

Regarding allantoin, in particular, activity of improving dehydration stress resistance in plants has been studied.

However, dehydration stress resistance is a feature different from high temperature stress resistance of a plant, and a mechanism of developing the former stress resistance is different from a mechanism of developing the latter stress resistance. Thus, there is no correlation between dehydration stress resistance and high temperature stress resistance. According to Non-Patent Document 3, for example, the DPB3-1/DREB2A interaction is necessary in order to induce high temperature stress resistance by DREB2A as described above. However, Non-Patent Document 3 describes that such interaction is not correlated with induction of dehydration stress resistance. As is apparent from the foregoing description, a certain component that is capable of improving resistance to stress other than high temperature stress, such as dehydration stress, is not always capable of improving high temperature stress resistance. On the basis of the finding of the past such that allantoin has activity of improving resistance to several types of stress other than high temperature stress of a plant, accordingly, it is not possible to predict activity of allantoin concerning high temperature stress resistance.

Meanwhile, the expression of DREB2A gene of a plant contributes to an improvement in resistance to various types of stress, such as high temperature stress resistance or dehydration stress resistance, as described above. Also, the expression of DREB2A gene is known to exert advantageous effects such as an improved rooting rate or prolonged life of cut flower. Through application of a substance capable of promoting the expression of DREB2A gene to a plant, advantageous effects as described above can be exerted. However, such substance was not provided in the past. While DREB2A-induced advantageous effects can be exerted in transgenic plants into which the DREB2A gene has been introduced as described in Patent Document 1, transgenic plants do not exist in nature, and industrial application thereof is difficult. Through application of a substance existing in nature to a plant, accordingly, a technique aimed at improvement in the expression of DREB2A gene in such plant is considered to be very useful.

Under the above circumstance, it is an object of the present invention to provide a means for improving high temperature stress resistance of a plant. It is another object of the present invention to provide a means for suppressing whitening of a plant.

### Solution to Problem

The present inventors found unexpected effects, such that high temperature stress resistance of a plant is improved, and whitening of a plant is suppressed, by allowing allantoin to act on the plant. This has led to the completion of the present invention. Specifically, the present invention includes the following.
(1) A method for improving the high temperature stress resistance of a plant, comprising a step of applying the high temperature stress resistance-improving agent comprising allantoin as an active ingredient to the plant.
(2) The method according to (1) above, wherein the step comprises applying the high temperature stress resistance-improving agent to the plant before the plant receives stress.
(3) A method for suppressing whitening of a plant due to high temperature stress, comprising a step of applying a whitening suppressor comprising allantoin as an active ingredient to a plant.
(4) Use of allantoin for improving the high temperature stress resistance of a plant.
(5). The use according to claim (4), for suppressing one or more selected from the group consisting of whitening of the plant due to high temperature stress, withering of the plant due to high temperature stress, and curling of plant leaves due to high temperature stress.
(6) Use of allantoin for suppressing whitening of a plant due to high temperature stress.

### Brief Description of Drawings

Fig. 1A illustrates compartments in a petri dish used in Experiment 1.
Fig. 1B shows photographs of petri dishes after *Arabidopsis thaliana* has been treated under various heat shock conditions and then grown for 1 week in Experiment 1.
Fig. 2 shows the viability of *Arabidopsis thaliana* treated under various heat shock conditions and then grown for 1 week in Experiment 1.
Fig. 3 shows photographs of onions 3 days after the initiation of heat shock treatment at 45°C for 1.5 hours in Experiment 2. In Fig. 3, the upper photograph shows onions of the allantoin treatment group (viability: 33.3%) and the lower photograph shows onions of the water treatment group (viability: 0%).
Fig. 4 shows photographs of *Brassica chinensis komatsuna* 7 days after the initiation of heat shock treatment at 45°C for 1 hour in Experiment 3. In Fig. 4, the upper photograph shows *Brassica chinensis komatsuna* of the allantoin treatment group (viability: 100%) and the lower photograph shows *Brassica chinensis komatsuna* of the water treatment group (viability: 33.3%).
Fig. 5A illustrates compartments in a petri dish used in Experiment 6.
Fig. 5B shows photographs of petri dishes in which *Arabidopsis thaliana* cultivated in media containing allantoin at various concentrations had been treated under heat shock conditions and under control conditions and grown for 1 week in Experiment 6.
Fig. 6 shows viability of *Arabidopsis thaliana* cultivated in media containing allantoin at various concentrations had been treated under heat shock conditions and under control conditions and grown for 1 week in Experiment 6.

### Description of Embodiments

### <1. Allantoin>

Allantoin is also referred to as "5-ureidohydantoin," and the free form thereof has a structure represented by the formula shown below.

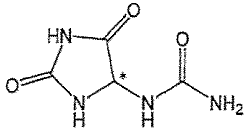

Allantoin has an asymmetric carbon (indicated by "*" in the formula), and it is either in the form of (R)-allantoin or (S)-allantoin. Allantoin used in the present invention may be (R)-allantoin or (S)-allantoin, or it may be in the form of a mixture of (R)-allantoin and (S)-allantoin.

Allantoin may be in the form applicable to a plant, and it may be a free form or an adequate form such as a solvate (e.g., a hydrate). Alternatively, it may be a mixture of two or more members of a free form, a hydrate, and the like.

### <2. Plants>

In the present invention, target plants to which allantoin, an allantoin-containing composition, a high temperature stress resistance-improving agent comprising allantoin as an active ingredient, a whitening suppressor comprising allantoin as an active ingredient, is to be applied are not particularly limited, and examples thereof include various plants, such as dicotyledons and monocotyledons.

Examples of dicotyledons to which allantoin, an allantoin-containing composition, a high temperature stress resistance-improving agent, a whitening suppressor, or a DREB2A gene expression promoter is to be applied include plants of *Pharbitis, Brassica, Convolvulus, Ipomoea, Arabidopsis thaliana, Cuscuta, Dianthus, Stellaria, Minuartia, Cerastium, Sagina japonica, Arenaria, Moehringia, Pseudostellaria, Honkenya, Spergula, Silene, Lychnis, Silene firma, Caryophyllaceae, Casuarinaceae, Saururaceae, Piperaceae, Chloranthaceae, Salicaceae, Myricaceae, Juglandaceae, Betulaceae, Fagaceae, Ulmaceae, Moraceae, Urticaceae, Podostemaceae, Proteaceae, Olacaceae, Santalaceae, Loranthaceae, Aristolochiaceae, Rafflesiaceae, Balanophoraceae, Polygonaceae, Chenopodiaceae, Amaranthaceae, Nyctaginaceae, Theligonaceae, Phytolaccaceae, Aizoaceae, Portulacaceae, Magnoliaceae, Trochodendraceae, Cercidiphyllaceae, Nymphaeaceae, Ceratophyllaceae, Ranunculaceae, Lardizabalaceae, Berberidaceae, Menispermaceae, Calycanthaceae, Lauraceae, Papaveraceae, Capparaceae, Brassicaceae, Droseraceae, Nepenthaceae, Crassulaceae, Saxifragaceae, Pittosporaceae, Hamamelidaceae, Platanaceae, Rosaceae, Leguminosae, Oxalidaceae, Geraniaceae, Linaceae, Zygophyllaceae, Rutaceae, Simaroubaceae, Meliaceae, Polygalaceae, Euphorbiaceae, Callitrichaceae, Buxaceae, Empetraceae, Coriariaceae, Anacardiaceae, Aquifoliaceae, Celastraceae, Staphyleaceae, Icacinaceae, Aceraceae, Hippocastanaceae, Sapindaceae, Sabiaceae, Balsaminaceae, Rhamnaceae, Vitaceae, Elaeocarpaceae, Tiliaceae, Malvaceae, Sterculiaceae, Dilleniaceae, Theaceae, Guttiferae, Elatinaceae, Tamaricaceae, Violaceae, Flacourtiaceae, Stachyuraceae, Passifloraceae, Begoniaceae, Cactaceae, Thymelaeaceae, Elaeagnaceae, Lythraceae, Punicaceae, Rhizophoraceae, Alangiaceae, Melastomataceae, Trapaceae, Onagraceae, Haloragaceae, Hippuridaceae, Araliaceae, Umbelliferae, Cornaceae, Diapensiaceae, Clethraceae, Pyrolaceae, Ericaceae, Myrsinaceae, Primulaceae, Plumbaginaceae, Ebenaceae, Symplocaceae, Styracaceae, Oleaceae, Buddlejaceae, Gentianaceae, Apocynaceae, Asclepiadaceae, Polemoniaceae, Boraginaceae, Verbenaceae, Lamiaceae, Solanaceae, Scrophulariaceae, Bignoniaceae, Pedaliaceae, Orobanchaceae, Gesneriaceae, Lentibulariaceae, Acanthaceae, Myoporaceae, Phrymaceae, Plantaginaceae, Rubiaceae, Caprifoliaceae, Adoxaceae, Valerianaceae, Dipsacaceae, Cucurbitaceae, Campanulaceae,* and *Asteraceae.*

Examples of monocotyledons to which allantoin, an allantoin-containing composition, a high temperature stress resistance-improving agent, a whitening suppressor, or a DREB2A gene expression promoter is to be applied include plants of *Spirodela, Lemna, Cattleya, Cymbidium, Dendrobium, Allium, Phalaenopsis, Vanda, Paphiopedilum, Orchidaceae, Typhaceae, Sparganiaceae, Potamogetonaceae, Najadaceae, Scheuchzeriaceae, Alismataceae, Hydrocharitaceae, Triuridaceae, Cyperaceae, Palmae, Araceae, Eriocaulaceae, Commelinaceae, Pontederiaceae, Juncaceae, Stemonaceae, Liliaceae, Amaryllidaceae, Dioscoreacea, Iridaceae, Musaceae, Zingiberaceae, Cannaceae,* and *Burmanniaceae.*

Target plants are not limited to wild-type plants, and mutants, transformants, and other type of plant may be the targets.

### <3. High temperature stress resistance-improving agent and method for improving high temperature stress resistance>

An aspect of the present invention concerns a high temperature stress resistance-improving agent for improving the high temperature stress resistance of a plant, comprising allantoin as an active ingredient.

An aspect of the present invention concerns a method for improving the high temperature stress resistance of a plant, comprising a step of applying the high temperature stress resistance-improving agent to a plant.

The term "high temperature stress" used herein refers to stress caused when a plant is exposed to a temperature higher than the general growth temperature, such as 25°C or higher, more specifically 30°C or higher, and further specifically 50°C or less. The duration during which a plant is exposed to such high temperature in a day is not particularly limited. For example, such duration can be 60 minutes or longer, and specifically 90 minutes or longer, and it can be 600 minutes or less.

The high temperature stress resistance-improving agent and the method for improving high temperature stress resistance of a plant according to the present invention are effective for improving resistance to high temperature stress. Such agent and method are particularly effective for improving resistance to stress that a plant receives when exposed to sufficient humidity and high temperature.

When a plant is exposed to a high temperature stress environment, in general, physiological damages, such as whitening (chlorosis) of a plant, withering of a plant, or curling of leaves, arise. With the use of the high temperature stress resistance-improving agent and the method for improving high temperature stress resistance of the present invention, one or more such physiological damages may be suppressed. As a result of the experiments described herein, among the physiological damages caused by high temperature stress, at least one of whitening of the plant due to high temperature stress (i.e., chlorosis), withering of the plant due to high temperature stress, and curling of plant leaves due to high temperature stress was found to be suppressed by the high temperature stress resistance-improving agent and the method for improving high temperature stress resistance of the present invention.

Regarding the high temperature stress resistance-improving agent and the method for improving high temperature stress resistance of the present invention, a mechanism of allantoin for improving high temperature stress resistance is not particularly limited. For example, a mechanism, such that high temperature stress resistance is improved by promoting the expression of DREB2A gene in the plant, is predicted. Promotion of the expression of DREB2A gene is described in detail in <5. DREB2A gene expression promoter and method of promoting the expression of DREB2A gene> below.

Regarding the high temperature stress resistance-improving agent and the method for improving high temperature stress resistance of the present invention, another mechanism of allantoin for improving high temperature stress resistance may be a mechanism, such that high temperature stress resistance is improved by promoting the expression of DREB2A gene in a plant and suppressing the expression of the heat shock transcription factor 3 (HSF3) gene. According to Non-Patent Document 1, in *Arabidopsis thaliana* in which DREB2A CA is overexpressed, the HSF3 gene expression level is increased, and high temperature stress resistance is then improved. According to the present invention, the DREB2A gene expression level is increased upon application of allantoin to a plant while the HSF3 gene expression level is suppressed and high temperature stress resistance is improved. Accordingly, allantoin is deduced to improve high temperature stress resistance of a plant due to a mechanism that is different from mechanisms that have been known in the past. Suppression of the expression of HSF3 gene is described in detail in <5. DREB2A gene expression promoter and method of promoting the expression of DREB2A gene> below.

The high temperature stress resistance-improving agent of the present invention may be any substance, provided that it contains allantoin and is capable of improving high temperature stress resistance of a plant. It may be allantoin or an allantoin-containing composition comprising allantoin and other components. The high temperature stress resistance-improving agent of the present invention can contain allantoin in an amount effective for improving high temperature stress resistance of a plant.

The high temperature stress resistance-improving agent of the present invention can be in any form, such as a solid or liquid.

When the high temperature stress resistance-improving agent of the present invention is an allantoin-containing composition, the composition may further contain other components useful for plants and components necessary for preparation of the agent, in addition to allantoin. Examples of other components include known fertilizer components. Examples of components necessary for preparation of the agent include carriers and liquid media.

When the high temperature stress resistance-improving agent of the present invention is an allantoin-containing composition, a method for producing the same is not particularly limited. For example, the agent can be produced by mixing components. In the case of a solid composition, according to need, operation, such as grinding, granulation, or dehydration, may be performed. In the case of a liquid composition, according to need, operation, such as stirring or dispersion, may be performed.

The method for improving the high temperature stress resistance of a plant of the present invention comprises a step of applying the high temperature stress resistance-improving agent to a plant. Target plants are required to have improved high temperature stress resistance. Examples thereof include plants that are cultivated under conditions in which plants may receive high temperature stress as described above. Specific plant species are described above.

A method of applying the high temperature stress resistance-improving agent of the present invention to a plant is not particularly limited, provided that the high temperature stress resistance-improving agent of the present invention or allantoin released from the high temperature stress resistance-improving agent of the present invention is brought into contact with a part of a plant, such as a root, stem, or leaf of a plant. The high temperature stress resistance-improving agent of the present invention may be directly applied to the plant, or the high temperature stress resistance-improving agent of the present invention may be applied to a cultivation medium, such as soil, in which the plant is fixed. In the present invention, the high temperature stress resistance-improving agent of the present invention can be applied to a plant in such a manner that allantoin is applied to the plant in an amount effective for improving high temperature stress resistance.

While the timing of application of the high temperature stress resistance-improving agent of the present invention to a plant is not particularly limited, the high temperature stress resistance-improving agent of the present invention is preferably applied to the plant before the plant receives high temperature stress. The high temperature stress resistance-improving agent of the present invention promotes the expression of DREB2A gene. As described above, expression of various stress resistant genes is deduced to be induced upon the expression of DREB2A gene. Accordingly, a plant to which the high temperature stress resistance-improving agent of the present invention has been applied before the plant receives high temperature stress is deduced to have achieved high temperature stress resistance, and the viability upon the plant receives high temperature stress is deduced to be high.

When the high temperature stress resistance-improving agent of the present invention is applied to the plant before the plant receives high temperature stress, the time point at which the high temperature stress resistance-improving agent of the present invention is applied to the plant is designated as "T1" and the time point at which exposure of the plant to high temperature stress is initiated is designated as "T2." In such a case, the period from T1 to T2 is preferably 0.5 to 10 days, more preferably 0.5 to 9 days, more preferably 0.5 to 8 days, more preferably 0.5 to 7 days, more preferably 0.5 to 6 days, more preferably 0.5 to 5 days, more preferably 0.5 to 4 days, more preferably 0.5 to 3 days, more preferably 0.5 to 2 days, and more preferably 0.5 to 1.5 days. According to this embodiment, the plant is exposed to high temperature stress while the high temperature stress resistance thereof has been improved through application of the high temperature stress resistance-improving agent of the present invention. Thus, viability after it is exposed to high temperature stress is high. The high temperature stress resistance-improving agent of the present invention may be applied to the plant the N number of times (N is 2 or greater) before the plant receives high temperature stress. In such a case, the period from T1ₙ (ₙ is an integer of 1 to N) at which the high temperature stress resistance-improving agent is applied to T2 is preferably within the range described above. Through application of the high temperature stress resistance-improving agent of the present invention a plurality of times, high temperature stress resistance of the plant can further be improved.

### <4. Whitening suppressor and method for suppressing plant whitening>

An aspect of the present invention concerns a whitening suppressor for suppressing whitening of a plant, comprising allantoin as an active ingredient.

An aspect of the present invention concerns a method for suppressing whitening of a plant, comprising a step of applying the whitening suppressor to a plant.

Whitening of a plant is also referred to as "chlorosis," and it is caused primarily by high temperature stress.

Target plants to be subjected to suppression of whitening in the present invention are plants that are required to undergo whitening suppression. Examples thereof include plants that are cultivated in the environment in which plants may be exposed to high temperature stress, such as the temperature conditions as described above. Specific plant species are described above.

The whitening suppressor of the present invention may be any substance, provided that it contains allantoin and is capable of suppressing whitening of a plant. It may be allantoin or an allantoin-containing composition comprising allantoin and other components. The whitening suppressor of the present invention can contain allantoin in an amount effective for whitening suppression of a plant.

The whitening suppressor of the present invention can be in any form, such as a solid or liquid.

When the whitening suppressor of the present invention is an allantoin-containing composition, such composition can be the same allantoin-containing composition as described with regard to the high temperature stress resistance-improving agent.

A method of applying the whitening suppressor of the present invention to a plant is not particularly limited, provided that the whitening suppressor of the present invention or allantoin released from the whitening suppressor of the present invention is brought into contact with a part of a plant, such as a root, stem, or leaf of a plant. The whitening suppressor of the present invention may be directly applied to the plant, or the whitening suppressor of the present invention may be applied to a cultivation medium, such as soil in which the plant is fixed. In the present invention, the whitening suppressor of the present invention can be applied to a plant in such a manner that allantoin is applied to the plant in an amount effective for whitening suppression.

While the timing of application of the whitening suppressor of the present invention to a plant is not particularly limited, the whitening suppressor of the present invention is preferably applied to the plant before the plant receives stress causing whitening (e.g., high temperature stress). The whitening suppressor of the present invention promotes the expression of DREB2A gene. As described above, expression of various stress resistant genes is deduced to be induced upon the expression of DREB2A gene. Accordingly, a plant to which the whitening suppressor of the present invention has been applied in advance before the plant receives stress is deduced to have achieved stress resistance, and whitening caused upon the plant receives stress is deduced to be suppressed effectively.

When the whitening suppressor of the present invention is applied to the plant before the plant receives stress causing whitening, the time point at which the whitening suppressor of the present invention is applied to the plant is designated as "T1" and the time point at which exposure of the plant to the stress is initiated is designated as "T2." In such a case, the period from T1 to T2 is preferably 0.5 to 10 days, more preferably 0.5 to 9 days, more preferably 0.5 to 8 days, more preferably 0.5 to 7 days, more preferably 0.5 to 6 days, more preferably 0.5 to 5 days, more preferably 0.5 to 4 days, more preferably 0.5 to 3 days, more preferably 0.5 to 2 days, and more preferably 0.5 to 1.5 days. According to this embodiment, the plant is exposed to the stress while the stress resistance thereof has been improved through application of the whitening suppressor of the present invention. Thus, whitening caused after the plant is exposed to the stress is suppressed more effectively. The whitening suppressor of the present invention may be applied to the plant the N number of times (N is 2 or greater)before the plant receives stress. In such a case, the period from T1ₙ (ₙ is an integer of 1 to N) at which the whitening suppressor is applied to T2 is preferably within the range described above. Through application of the whitening suppressor of the present invention a plurality of times, whitening of a plant can be suppressed more effectively.

### <5. DREB2A gene expression promoter and method of promoting the expression of DREB2A gene>

An aspect of the present disclosure concerns a DREB2A gene expression promoter for promoting the expression of DREB2A gene in a plant, comprising allantoin as an active ingredient. The scope of protection sought is that defined by the appended claims.

An aspect of the present disclosure concerns a method of promoting the expression of DREB2A gene in the plant, comprising a step of applying the DREB2A gene expression promoter to a plant.

These aspects of the present disclosure make contributions to improving resistance to various types of stress, such as high temperature stress resistance or dehydration stress resistance, by promoting the expression of DREB2A gene in a plant. These aspects also exert advantageous effects, such as an improved rooting rate or a prolonged life of cut flower, of a target plant.

According to the method of promoting the expression of DREB2A gene in the plant of the present disclosure, preferably, the expression of HSF3 gene is suppressed.

In the present disclosure, a process of mRNA expression using genomic DNA as a template (i.e., transcription) and/or a process of protein synthesis using the mRNA as a template (i.e., translation) are within the scope of "gene expression." The term "gene" used in the present disclosure refers to a nucleic acid comprising a nucleotide sequence encoding a particular polypeptide, and the term typically refers to genomic DNA of a plant or mRNA generated with the use of genomic DNA as a template.

When the expression of DREB2A gene is promoted in the present disclosure, the DREB2A gene expression level is increased to a significant extent, compared with a plant to which allantoin has not been applied. Whether or not the expression of DREB2A gene is promoted in a plant can be inspected by detecting an increase in the level of mRNA encoding a DREB2A polypeptide and/or an increase in the level of the DREB2A polypeptide in a plant. An extent of promotion of the expression of DREB2A gene is not particularly limited in the present disclosure. When the DREB2A gene expression level in a plant to which the DREB2A gene expression promoter of the present disclosure has not been applied is designated as 100, the DREB2A gene expression level is, for example, 120 or higher, preferably 150 or higher, and more preferably 200 or higher in a plant to which the DREB2A gene expression promoter of the present disclosure has been applied.

When the expression of HSF3 gene is suppressed in the present invention, the HSF3 gene expression level is decreased to a significant extent, compared with a plant to which allantoin is not applied. Whether or not the expression of HSF3 gene is suppressed in a plant can be inspected by detecting a decrease in the level of mRNA encoding the HSF3 polypeptide and/or a decrease in the level of the HSF3 polypeptide in a plant. An extent of suppression of the expression of HSF3 gene is not particularly limited in the present invention. When the HSF3 gene expression level in a plant to which the DREB2A gene expression promoter of the present disclosure has not been applied is designated as 100, the HSF3 gene expression level is, for example, 90 or lower, preferably 80 or lower, and more preferably 75 or lower in a plant to which the DREB2A gene expression promoter of the present disclosure has been applied.

Target plants to be subjected to promotion of the expression of DREB2A gene in the present disclosure are plants that are required to undergo gene expression promotion. Examples thereof include plants that are cultivated in the environment in which plants may be exposed to high temperature stress, such as the temperature conditions as described above, and other stress. Specific plant species are described above.

The DREB2A gene expression promoter of the present disclosure may be any substance, provided that it contains allantoin and is capable of promoting the expression of DREB2A gene in a plant. It may be allantoin or an allantoin-containing composition comprising allantoin and other components. The DREB2A gene expression promoter of the present disclosure can contain allantoin in an amount effective for promoting the expression of DREB2A gene in the plant. The DREB2A gene expression promoter of the present disclosure can also contain allantoin in an amount effective for suppression of the expression of HSF3 gene.

The DREB2A gene expression promoter of the present disclosure can be in any form, such as a solid or liquid.

When the DREB2A gene expression promoter of the present disclosure is an allantoin-containing composition, such composition can be the same allantoin-containing composition as described with regard to the high temperature stress resistance-improving agent.

A method of applying the DREB2A gene expression promoter of the present disclosure to a plant is not particularly limited, provided that the DREB2A gene expression promoter of the present disclosure or allantoin released from the DREB2A gene expression promoter of the present invention is brought into contact with a part of a plant, such as a root, stem, or leaf of a plant. The DREB2A gene expression promoter of the present disclosure may be directly applied to the plant, or the DREB2A gene expression promoter of the present disclosure may be applied to a cultivation medium, such as soil in which the plant is fixed. In the present disclosure, the DREB2A gene expression promoter of the present disclosure can be applied to a plant in such a manner that allantoin is applied to the plant in an amount effective for promoting the expression of DREB2A gene in the plant. In the present disclosure, the DREB2A gene expression promoter of the present disclosure can be applied to a plant in such a manner that allantoin is applied to the plant in an amount effective for suppressing the expression of HSF3 gene in the plant.

While the timing of application of the DREB2A gene expression promoter of the present disclosure to a plant is not particularly limited, the DREB2A gene expression promoter of the present disclosure is preferably applied to the plant before the plant receives stress (e.g., high temperature stress). The DREB2A gene expression promoter of the present disclosure promotes the expression of DREB2A gene. As described above, the expression of various stress resistant genes may be induced upon the expression of DREB2A gene. Accordingly, a plant to which the DREB2A gene expression promoter of the present disclosure has been applied in advance before the plant receives stress is deduced to have achieved stress resistance, and viability thereof upon the plant receives stress is deduced to be high.

When the DREB2A gene expression promoter of the present disclosure is applied to the plant before the plant receives stress, the time point at which the DREB2A gene expression promoter of the present disclosure is applied to the plant is designated as "T1" and the time point at which exposure of the plant to the stress is initiated is designated as "T2." In such a case, the period from T1 to T2 is preferably 0.5 to 10 days, more preferably 0.5 to 9 days, more preferably 0.5 to 8 days, more preferably 0.5 to 7 days, more preferably 0.5 to 6 days, more preferably 0.5 to 5 days, more preferably 0.5 to 4 days, more preferably 0.5 to 3 days, more preferably 0.5 to 2 days, and more preferably 0.5 to 1.5 days. According to this embodiment, the plant is exposed to the stress while the stress resistance thereof has been improved through application of the DREB2A gene expression promoter of the present disclosure. Thus, viability after the plant is exposed to the stress is high. The DREB2A gene expression promoter of the present disclosure may be applied to the plant the N number of times (N is 2 or greater) before the plant receives stress. In such a case, the period from T1ₙ (ₙ is an integer of 1 to N) at which the DREB2A gene expression promoter is applied to T2 is preferably within the range described above. Through application of the DREB2A gene expression promoter of the present disclosure a plurality of times, stress resistance of the plant can further be improved.

In the present invention, genes comprising nucleotide sequences encoding polypeptides annotated as "dehydration-responsive element-binding protein 2A" or "dehydration-responsive element-binding protein 2A-like" in the database provided by the National Center for Biotechnology Information (NCBI) (http://www.ncbi.nlm.nih.gov/) or other databases and genes comprising nucleotide sequences encoding polypeptides having functions homologous to the polypeptides described above are within the scope of the DREB2A gene.

The genes comprising nucleotide sequences encoding polypeptides annotated as "dehydration-responsive element-binding protein 2A" or "dehydration-responsive element-binding protein 2A-like" in the database provided by NCBI (http://www.ncbi.nlm.nih.gov/) can be confirmed on the basis of the results of search demonstrated in the website at the URL indicated below: http://www.ncbi.nlm.nih.gov/gene/?term=dehydration-responsive+element-binding+protein+2A.

For example, the nucleotide sequence of cDNA of the DREB2A gene of *Arabidopsis thaliana* (AGI code: AT5G05410) is as shown in SEQ ID NO: 1. A partial nucleotide sequence in the nucleotide sequence as shown in SEQ ID NO: 1 (i.e., a region from nucleotides 189 to 1196) is a region encoding the DREB2A polypeptide.

In some target plants, expression of a gene comprising a nucleotide sequence encoding a polypeptide having functions homologous to a polypeptide annotated as "dehydration-responsive element-binding protein 2A" or "dehydration-responsive element-binding protein 2A-like" may be promoted. Examples of genes comprising nucleotide sequences encoding polypeptides having functions homologous to the polypeptides annotated as dehydration-responsive element-binding protein 2A or dehydration-responsive element-binding protein 2A-like include, but are not limited to:
(1) a gene comprising a nucleotide sequence encoding a polypeptide comprising an amino acid sequence derived from the amino acid sequence of the DREB2A polypeptide of *Arabidopsis thaliana* encoded by a partial nucleotide sequence at positions 189 to 1196 in the nucleotide sequence as shown in SEQ ID NO: 1 by substitution, deletion, insertion, and/or addition of one or several, such as 1 to 20, preferably 1 to 15, more preferably 1 to 10, still more preferably 1 to 5, further preferably 1 to 3, and still further preferably 1 or 2 amino acids and the polypeptide having functions homologous to the DREB2A polypeptide of *Arabidopsis thaliana,* and
(2) a gene comprising a nucleotide sequence encoding a polypeptide comprising an amino acid sequence having sequence identity of 60% or higher, preferably 70% or higher, more preferably 80% or higher, still more preferably 85% or higher, further preferably 90% or higher, still further preferably 95% or higher, and furthermore preferably 98% or higher to the amino acid sequence of the DREB2A polypeptide of *Arabidopsis thaliana* and the polypeptide having functions equivalent to the DREB2A polypeptide of *Arabidopsis thaliana.*

In the present invention, amino acid sequence identity can be determined with the use of, for example, techniques or sequence analysis software well known in the art. The term "amino acid sequence identity" refers to the proportion (%) of the number of consistent amino acid residues relative to the total number of amino acid residues (including the number of gaps when gaps are inserted), when, for example, the amino acid sequence of the DREB2A polypeptide of *Arabidopsis thaliana* and another amino acid sequence are aligned by inserting gaps, according to need, so as to maximize the degree of consistency between these two amino acid sequences, and amino acid sequence identity can be determined using protein search systems, such as BLAST or FASTA (Karlin, S. et al., 1993, Proceedings of the National Academic Sciences, U.S.A., Vol. 90, pp. 5873-5877; Altschul, S. F. et al., 1990, Journal of Molecular Biology, Vol. 215, pp. 403-410; Pearson, W. R. et al., 1988, Proceedings of the National Academic Sciences, U.S.A., Vol. 85, pp. 2444-2448).

In the present invention, genes comprising nucleotide sequences encoding polypeptides annotated as "heat stress transcription factor A-1b" or "heat stress transcription factor A-1b-like" in the database provided by the National Center for Biotechnology Information (NCBI) (http://www.ncbi.nlm.nih.gov/) or other databases and genes comprising nucleotide sequences encoding polypeptides having functions homologous to the polypeptides described above are within the scope of the HSF3 gene.

The genes comprising nucleotide sequences encoding polypeptides annotated as "heat stress transcription factor A-1b" or "heat stress transcription factor A-1b-like" in the database provided by NCBI (http://www.ncbi.nlm.nih.gov/) can be confirmed on the basis of the results of search demonstrated in the website at the URL indicated below: http://www.ncbi.nlm.nih.gov/gene/?term=heat+stress+transcription+factor+A-1b.

For example, the nucleotide sequence of cDNA of the HSF3 gene of *Arabidopsis thaliana* (AGI code: AT5G16820) is as shown in SEQ ID NO: 2. A partial nucleotide sequence at positions 174 to 1619 in the nucleotide sequence as shown in SEQ ID NO: 2 is a region encoding the HSF3 polypeptide.

In some target plants, expression of a gene comprising a nucleotide sequence encoding a polypeptide having functions homologous to a polypeptide annotated as "heat stress transcription factor A-1b" or "heat stress transcription factor A-1b-like" may be suppressed.

Examples of genes comprising nucleotide sequences encoding polypeptides having functions homologous to the polypeptides annotated as "heat stress transcription factor A-1b" or "heat stress transcription factor A-1b-like" include, but are not limited to:
(1) a gene comprising a nucleotide sequence encoding a polypeptide comprising an amino acid sequence derived from the amino acid sequence of the HSF3 polypeptide of *Arabidopsis thaliana* encoded by a partial nucleotide sequence at positions 174 to 1619 in the nucleotide sequence as shown in SEQ ID NO: 2 by substitution, deletion, insertion, and/or addition of one or several, such as 1 to 20, preferably 1 to 15, more preferably 1 to 10, still more preferably 1 to 5, further preferably 1 to 3, and still further preferably 1 or 2 amino acids and the polypeptide having functions homologous to the HSF3 polypeptide of *Arabidopsis thaliana,* and
(2) a gene comprising a nucleotide sequence encoding a polypeptide comprising an amino acid sequence having sequence identity of 60% or higher, preferably 70% or higher, more preferably 80% or higher, still more preferably 85% or higher, further preferably 90% or higher, still further preferably 95% or higher, and furthermore preferably 98% or higher to the amino acid sequence of the HSF3 polypeptide of *Arabidopsis thaliana* and the polypeptide having functions equivalent to the HSF3 polypeptide of *Arabidopsis thaliana.*

### Examples

Hereafter, the present invention is described with reference to specific examples described below.

### <Experiment 1. Evaluation of high temperature stress resistance of Arabidopsis thaliana in which allantoin is accumulated>

### 1. Plant material

The 3 lines of *Arabidopsis thaliana L. Heynh.,* accession Columbia-0 with different genetic backgrounds indicated below were used.
(1) Wild-type line (WT)
(2) Allantoinase gene-deletion line (aln-1)
(3) Allantoinase gene-deletion line complemented with allantoinase gene (aln-1 35S:ALN)

These plants are the same as those used in Non-Patent Document 4. The complemented line of the allantoinase gene-deletion line (aln-1 35S:ALN) is the same as the line indicated as "35Spro:ALN/aln-1" in Non-Patent Document 4.

The allantoinase gene-deletion line of (2) above (SALK_000325, Yang, J. and Han K.-H, Plant Physiology, 134: 1039-1049) was obtained from the Arabidopsis Biological Resource Center (Ohio State University).

The complemented line of the allantoinase gene-deletion line of (3) above (aln-1 35S:ALN) was obtained by introducing DNA encoding the full-length sequence of allantoinase derived from the wild-type *Arabidopsis thaliana* line into the aln-1 using a vector. A specific method of preparation thereof is as described in Non-Patent Document 4.

### 2. Culture medium

A salt mixture for the half-strength Murashige & Skoog medium (Murashige T., F. Skoog F. 1962, A revised medium for rapid growth and bioassays with tobacco tissue cultures, Physiologia Plantarum 15: 473-497), sucrose, vitamins, and gellan gum (solidifier) were dissolved in MES (2-(N-morpholino)ethanesulfonic acid) buffer. The solution was autoclaved, dispensed in amounts of 25 ml each into a deep sterilized petri dish FX (90 × 20 mm, Sansei Medical Co., Ltd.), and allowed to solidify in the clean bench to obtain 1/2 MS solid medium. The resulting 1/2 MS solid medium contains the components at concentrations shown in Table 1.

**Table 1**

| Composition | Final concentration |
|---|---|
| Salt mixture for Murashige & Skoog medium (Wako Pure Chemical Industries, Ltd.) | 2.3 g/l |
| Sucrose (Wako Pure Chemical Industries, Ltd.) MS vitamins | 10 g/l ** |
| MES (50 g/l, pH 5.7 with KOH) | 10 ml/l |
| Gellan gum (Wako Pure Chemical Industries, Ltd.) | 4 g/l |

| | |
|---|---|
| **The final concentration of MS vitamins is as follows: 0.25 mg/l thiamine hydrochloride; 0.25 mg/l nicotinic acid; 0.25 mg/l pyridoxine hydrochloride; 1 mg/l glycine; and 50 mg/l myo-inositol. A stock solution of the composition at a concentration 500 times greater than that was used for preparing the medium. | |

### 3. Growth conditions and high temperature stress treatment

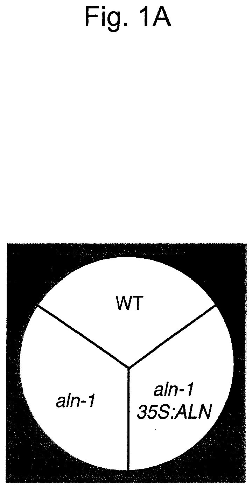

(1) Several hundreds of mature seeds of the plant were introduced into a 1.5-ml tube. The treatments (2) to (6) described below were carried out in the clean bench.
(2) 2.5% (v/v) sodium hypochlorite (1 ml) was introduced into the tube containing the seeds, mounted on a small rotation mixer (18 rpm), and sterilized for 10 minutes.
(3) Following spinning-down, the sodium hypochlorite solution was discarded, 1 ml of sterile water was added, and the treatment (2) was carried out.
(4) The treatment (3) was repeated 3 times with the use of sterile water to thoroughly wash the seeds.
(5) A petri dish filled with 1/2 MS solid medium was radially divided into 3 compartments, and 9 seeds of each line were inoculated into each compartment (27 seeds in total in a petri dish) (Fig. 1A).
(6) The petri dish was introduced into the clean bench while keeping the lid open, water in the vicinity of the seeds was allowed to evaporate (for 20 to 30 minutes), and the petri dish was then sealed with a surgical tape.
(7) Each petri dish was wrapped with aluminum foil and subjected to low-temperature treatment (4°C) for 2 days for dormancy breaking.
(8) The resultant was transferred to a culture chamber and cultured therein at 22°C under long-day conditions (light application for 16 hours under fluorescent light: 0.07 mmol photons m⁻¹ s⁻¹) for 7 days.
(9) The 7-day-old plants aseptically grown in (8) were introduced into an incubator preset to 45°C, and heat shock was applied in the dark for 75, 90, or 105 minutes. A control test was carried out by continuously growing plants at 22°C without the application of heat shock.
(10) After the heat shock treatment, the petri dish was cooled in an incubator at 22°C for 10 to 15 minutes, and plants were allowed to grow again under the conditions described in (8) for another 1 week. In case of serious damage, a phenomenon of chlorosis (whitening) of leaves would become observable approximately 3 days after the initiation of the test. The viability was evaluated on the basis of such phenomenon.

The test described above was carried out two times.

### 4. Results

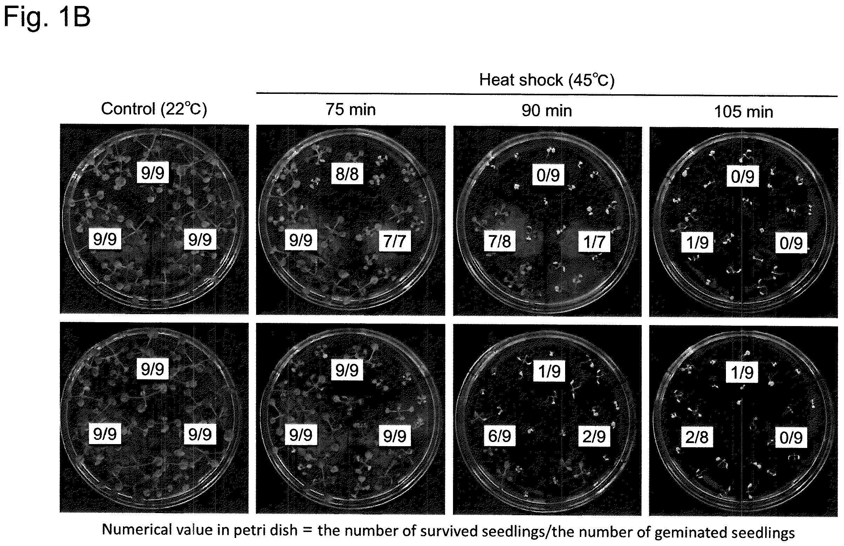

Fig. 1B shows photographs of petri dishes after plants had been treated under various heat shock conditions and grown for 1 week. Plant line positions in each petri dish shown in Fig. 1B are as shown in Fig. 1A. Each numerical value in the petri dish indicates the ratio of "the number of survived seedlings" relative to "the number of geminated seedlings" (the number of survived seedlings/the number of geminated seedlings) in each compartment.

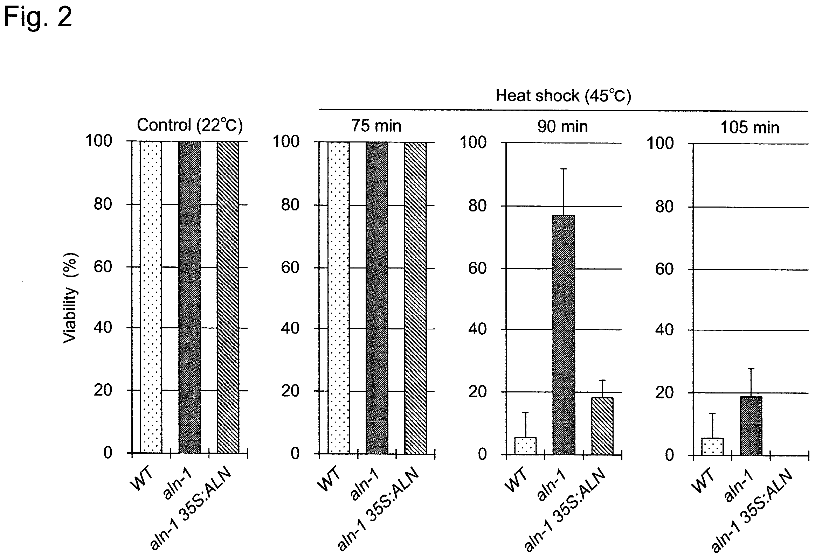

Fig. 2 shows the viability under various heat shock conditions. The viability shown in Fig. 2 is the value obtained by determining the viability (%) under various conditions based on the number of survived seedlings indicated in Fig. 1B and calculating the average of two tests.

As is apparent from the test results shown in Fig. 1B and Fig. 2, the allantoinase gene-deletion line (aln-1) has resistance to high temperature stress. Since allantoin is not metabolized and is accumulated in the allantoinase gene-deletion line (aln-1), it was concluded that the high temperature stress resistance observed in this experiment was achieved by the presence of allantoin at high concentration in the plant body.

A phenomenon of chlorosis (whitening) of leaves was observed in plants damaged by high temperature stress.

### <Experiment 2. Provision of high temperature stress resistance to monocotyledons via allantoin application>

A 5-cm polypot was mounted on a balance dish BD-2 (AS ONE Corporation), 70 ml of vermiculite (Protoleaf Inc.) was introduced thereinto, 80 ml of culture soil (TAKII & CO., LTD.) was further introduced thereinto, 3 seeds of onion (variety: Neoearth; TAKII & CO., LTD.) were sowed at 3 positions, and 20 ml of vermiculite was further introduced thereinto, so as to cover the soil. Thereafter, 50 ml of tap water was supplied to the balance dish two times. The polypot (together with the balance dish) was mounted on a bat, and the resultant was then introduced into an incubator at 22°C and 10,000 Lux for a light period of 12 hours and a dark period of 12 hours, so as to initiate cultivation. The day on which cultivation was initiated was designated as Day 0 after sowing. Germination was observed 5 days after sowing. While leaving 3 plants in each pot (i.e., a plant in each position), other plants were removed therefrom 6 days after sowing.

Tap water or an aqueous solution of 1 mM allantoin was applied to each test group (40 ml/pot) 6 days after sowing (hereafter, referred to as the "water treatment group" or the "allantoin treatment group," respectively). Tap water was applied to all the pots (40 ml/pot) 11 days after sowing. The pots mounted on the bats were introduced into the incubator 13 days after sowing (at the 2-true-leaf stage in the case of onions), and the plants were exposed to high temperature stress at 45°C for 1 hour, 1.5 hours, and 2 hours. Soil in the pots was sufficiently humidified before and after high temperature stress application. After the completion of the high temperature stress treatment, the temperature in the incubator was cooled to 22°C, and cultivation was continued. Water was supplied 2 days after the heat shock treatment (40 ml/pot). Thereafter, water was supplied in an amount of 40 ml per pot at intervals of 2 days.

The viability 3 days after the heat shock treatment was as follows. The viability achieved by the 1-hour treatment of the water treatment group and of the allantoin treatment group was 22.2% and 66.7%, respectively, the viability achieved by the 1.5-hour treatment was 0% and 33.3%, respectively, and the viability achieved by the 2-hour treatment of the water treatment group and of the allantoin treatment group was 0% and 11.1%, respectively. The same viability was observed 8 days after heat shock treatment. Accordingly, the effects of imparting high temperature stress resistance via allantoin application were verified.

The "viability" indicates the proportion of the number of survived plants relative to the number of plants subjected to the heat shock treatment. The viability was calculated by regarding plants that would no longer grow (i.e., the plants with the youngest leaves suffering from physical damage, such as whitening, withering, or curling of leaves) as unsurvived plants.

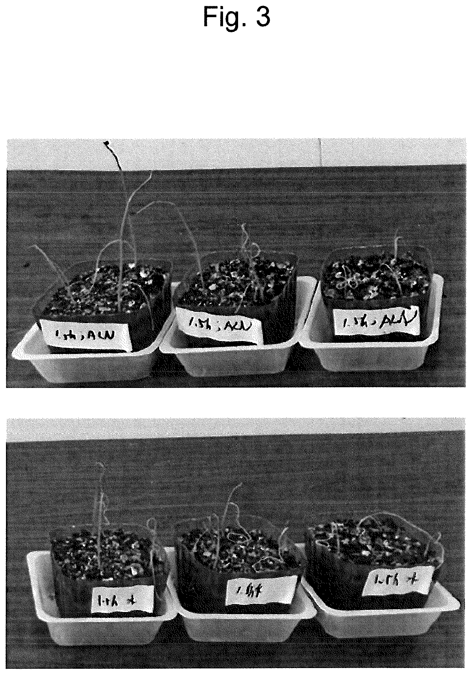

Fig. 3 shows photographs of plants subjected to heat shock treatment at 45°C for 1.5 hours and then grown for 3 days. In Fig. 3, the upper photograph shows plants of the allantoin treatment group (viability: 33.3%) and the lower photograph shows plants of the water treatment group (viability: 0%).

### <Experiment 3. Provision of high temperature stress resistance to dicotyledons via allantoin application>

A 5-cm polypot was mounted on a balance dish BD-2 (AS ONE Corporation), 70 ml of vermiculite (Protoleaf Inc.) was introduced thereinto, 80 ml of culture soil (TAKII & CO., LTD.) was further introduced thereinto, 3 seeds of *Brassica chinensis komatsuna* (variety: Rakuten, TAKII & CO., LTD.) were sowed at the center of the pot, and 20 ml of vermiculite was further introduced thereinto, so as to cover the soil. Thereafter, 50 ml of tap water was supplied to the balance dish two times. The polypot (together with the balance dish) was mounted on a bat and introduced into an incubator at 22°C and 10,000 Lux for a light period of 12 hours and a dark period of 12 hours, so as to initiate cultivation. The day on which cultivation was initiated was designated as Day 0 after sowing.

Germination was observed 3 days after sowing. While leaving one plant in each pot, other plants were removed therefrom 6 days after sowing. Tap water or an aqueous solution of 1 mM allantoin was applied to each test group (40 ml/pot) 6 days after sowing. Tap water was applied to all the pots (40 ml/pot) 11 days after sowing. Tap water or an aqueous solution of 1 mM allantoin was applied to each test group (40 ml/pot) 13 days after sowing. The pots mounted on the bats were introduced into the incubator 14 days after sowing (at the 2-true-leaf stage in the case of *Brassica chinensis komatsuna*), and the plants were exposed to high temperature stress at 45°C for 1 hour. Soil in the pots was sufficiently humidified before and after high temperature stress application. After the completion of the high temperature stress treatment, the temperature in the incubator was cooled to 22°C, and cultivation was continued. Water was supplied 2 days after the heat shock treatment (40 ml/pot). Thereafter, water was supplied in an amount of 40 ml per pot at intervals of 2 days.

The viability 7 days after the heat shock treatment was as follows. The viability achieved by the 1-hour treatment of the water treatment group and of the allantoin treatment group was 33.3% and 100%, respectively. Accordingly, the effects of imparting high temperature stress resistance via allantoin application were verified.

The "viability" indicates the proportion of the number of survived plants relative to the number of plants subjected to the heat shock treatment. The viability was calculated by regarding plants that would no longer grow (i.e., the plants with the youngest leaves suffering from physical damage, such as whitening, withering, or curling of leaves) as unsurvived plants.

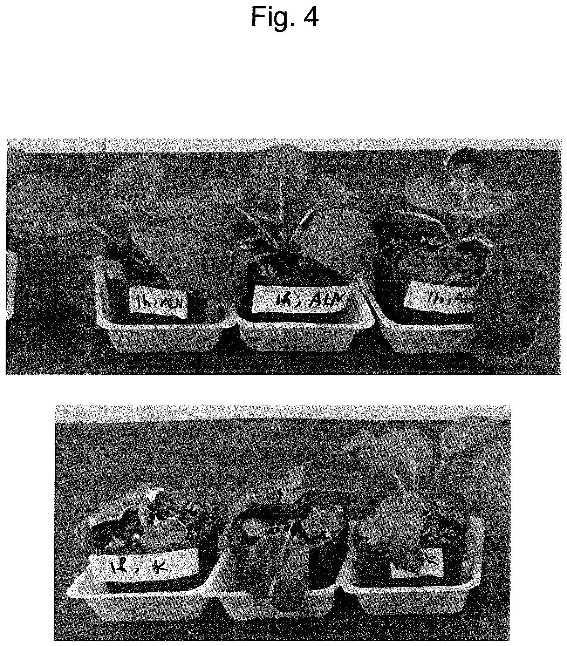

Fig. 4 shows photographs of plants subjected to heat shock treatment at 45°C for 1 hours and then grown for 7 days. In Fig. 4, the upper photograph shows plants of the allantoin treatment group (viability: 100%) and the lower photograph shows plants of the water treatment group (viability: 33.3%).

### <Experiment 4. Promotion of the expression of DREB2A gene in plants in which allantoin is accumulated>

### 1. Summary

In order to precisely inspect the influence of allantoin imposed on gene expression of *Arabidopsis thaliana L. Heynh.,* raw microarray data of the aln-1 mutant that accumulates allantoin as a result of allantoinase gene deletion (NCBI Gene Expression Omnibus accession number GSE44922) was subjected to normalization in accordance with the technique described in Konishi, T., 2004, Three-parameter long normal distribution ubiquitously found in cDNA microarray data and its application to parametric data treatment. BMC Bioinformatics 5: 5. The normalized data was subjected to two-way analysis of variance, the results of analysis were compared with those of wild-type line under strict conditions (P < 0.001), and the genes whose transcript levels varied by 3-fold or more were selected (Konishi, T., 2011, Microarray test results should not be compensated for multiplicity of gene contents, BMC Syst. Biol. 5 (Suppl 2): S6). These genes were subjected to gene ontology (GO) biological process analysis with the use of VirtualPlant (version 1.3; http://virtualplant.bio.nyu.edu/cgi-bin/vpweb/).

### 2. Procedures in detail

Total RNA was extracted from seedlings of 2-week-old wild-type *Arabidopsis thaliana* line and the aln-1 mutant. Two independent biological samples were used for each genotype. Reverse transcription of RNA into cDNA, labeling thereof, and hybridization thereof to Affymetrics ATH1 Gene Chips were carried out in accordance with the manufacturer's instructions. After the microarrays subjected to hybridization were washed, signals derived from hybridization were collected using the Affymetrics GeneChip Scanner 3000 7G. Thus, raw data from 4 microarrays; i.e., raw data from 2 microarrays of wild-type line and of the aln-1 mutant, were obtained in total. Such procedure is described in Watanabe, S. et al., 2014, Plant Cell Environ., 37: 1022-1036.

Such raw data from microarrays are registered and disclosed under accession numbers of the NCBI Gene Expression Omnibus (http://www.ncbi.nlm.nih.gov/geo/). Such microarray data were normalized by the parametric method using the 3-parameter lognormal distribution model using the SuperNORM service provided by Skylight Biotech, Inc., and the gene expression levels were converted into z-scores (Konishi, T., 2004, as described above). As a result of normalization, gene expression levels between different microarrays can become comparable. The normalized data are registered under Accession Number: GSE73841.

The gene expression levels were compared between wild-type line and the aln-1 mutant. The genes whose expression levels were increased or decreased by at least 3 times in the aln-1 mutant were selected via the two-way analysis of variance (ANOVA) under strict test conditions (P < 0.001) in accordance with the method of Konishi, 2011. With the use of the VirtualPlant (version 1.3; http://virtualplant.bio.nyu.edu/cgi-bin/vpweb/) BioMaps tools with default setting (the Fisher's exact test with false discovery rate correction, P < 0.01), gene ontology (GO) biological process analysis was carried out in accordance with the Arabidopsis genomic annotation (TAIR release 10; http://arabidopsis.org/).

### 3. Results

As a results of the analysis, the expression level of the DREB2A gene (AGI code: AT5G05410; SEQ ID NO: 1) in the aln-1 mutant line was found to be 3.17 times greater than that in wild-type line.

In contrast, the expression level of the heat shock transcription factor 3 gene (HSF3) (AGI code: AT5G16820, SEQ ID NO: 2) in the aln-1 mutant was found to be 0.577 times greater than that in wild-type line.

### <Experiment 5. Promotion of the expression of DREB2A gene via cultivation in allantoin-containing medium>

Seeds of wild-type *Arabidopsis thaliana* line (Columbia-0) were sterilized in 2.5% (v/v) sodium hypochlorite, sowed in the 1/2 MS solid medium described in Experiment 1 (Table 1) (the medium was supplemented with 1 mM allantoin; allantoin was not added to the control group; a deep petri dish with a diameter of 90 mm and a height of 20 mm), and then allowed to grow under long-day conditions (in an incubator at 5,000 Lux, a light period of 16 hours and a dark period of 8 hours, 22°C) for 14 days. With the use of a commercialized kit, RNA extraction from leaves and stems of *Arabidopsis thaliana* (NucleoSpin RNAII, MACHEREY-NAGEL), reverse transcription (ReverTra Ace qPCR RT Master Mix, TOYOBO), and quantitative PCR (KAPA SYBR FAST qPCR Master Mix, KAPABIOSYSTEMS) were carried out. As a reference gene, ACT2 (AGI code: AT3G18780) was used. As a result, the relative expression level of DREB2A (AGI code: AT5G05410; SEQ ID NO: 1) and that of HSF3 (AGI code: AT5G16820; SEQ ID NO: 2) were 2.2 times and 0.7 times greater than the expression level of the control group, respectively.

### <Experiment 6. Evaluation of high temperature stress resistance of Arabidopsis thaliana cultivated in allantoin-containing medium>

### 1. Plant material

The wild-type (WT) line of *Arabidopsis thaliana (L.) Heynh.,* accession Columbia-0 described in Experiment 1 was used.

### 2. Culture medium

The 1/2 MS solid medium described in Experiment 1 (Table 1) was used (10 µM, 100 µM, or 1,000 µM allantoin was further added to the allantoin-supplemented group, allantoin was not added to the allantoin-free group, a deep sterilized petri dish No. 903 VALMARK; Ina-Optika Corporation).

### 3. Growth conditions and high temperature stress treatment

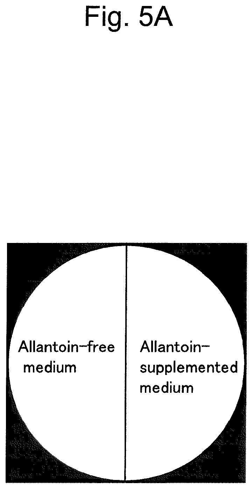

(1) Several hundreds of mature seeds of the plant were introduced into a 1.5-ml tube. The treatments (2) to (6) described below were carried out in the clean bench.
(2) 2.5% (v/v) sodium hypochlorite (1 ml) was introduced into the tube containing seeds, mounted on a small rotation mixer (18 rpm), and sterilized for 10 minutes.
(3) Following spinning-down, the sodium hypochlorite solution was discarded, 1 ml of sterile water was added, and the treatment (2) was carried out.
(4) The treatment (3) was repeated 3 times with the use of sterile water to thoroughly wash the seeds.
(5) In the petri dish, a circular accommodation part (a plane view) is divided into 2 semicircular compartments by a partition wall extended toward a diametrical direction. The allantoin-free 1/2 MS solid medium was accommodated in one of the compartments of the petri dish, the allantoin-supplemented 1/2 MS solid medium was accommodated in another compartment, and 15 seeds were sowed in each compartment (i.e., 30 seeds in total per petri dish) (Fig. 5A).
(6) The petri dish was introduced into the clean bench while keeping the lid open, water in the vicinity of the seeds was allowed to evaporate (for 20 to 30 minutes), and the petri dish was then sealed with a surgical tape.
(7) Each petri dish was wrapped with aluminum foil and subjected to low-temperature treatment (4°C) for 2 days for dormancy breaking.
(8) The resultant was transferred to a culture chamber and cultured therein at 22°C under long-day conditions (light application for 16 hours under fluorescent light: 0.07 mmol photons m⁻¹ s⁻¹) for 7 days.
(9) The 7-day-old plants aseptically grown in (8) were introduced into an incubator preset to 45°C, and heat shock was applied in the dark for 105 minutes. A control test was carried out by continuously growing plants at 23°C without the application of heat shock.
(10) Thereafter, the petri dish was cooled in an incubator at 23°C for 10 to 15 minutes, and the plants were then allowed to grow again for 1 week under the conditions (8). In case of serious damage, a phenomenon of chlorosis (whitening) of leaves would become observable approximately 3 days after the initiation of the test. The viability was evaluated on the basis of such phenomenon.

The test described above was carried out two times.

### 4. Results

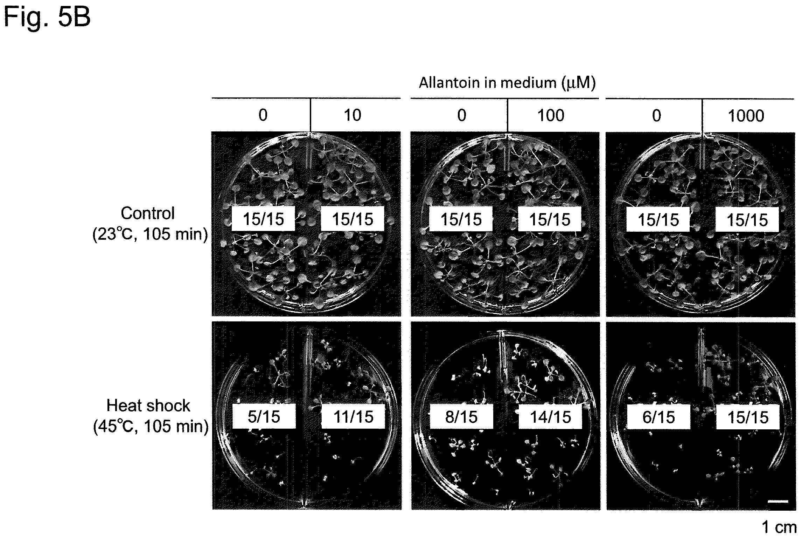

Fig. 5B shows photographs of petri dishes after *Arabidopsis thaliana* had been treated under various heat shock conditions and then grown for 1 week. Plant line positions in each petri dish shown in Fig. 5B are as shown in Fig. 5A. Each numerical value in the petri dish indicates the ratio of "the number of survived seedlings" relative to "the number of geminated seedlings" (the number of survived seedlings/the number of geminated seedlings) in each compartment.

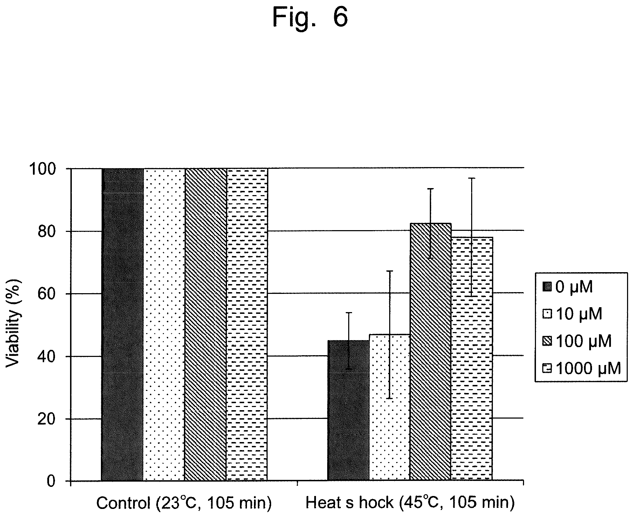

Fig. 6 shows viability under various heat shock conditions. The viability shown in Fig. 6 is the value obtained by determining the viability (%) under various conditions based on the number of survived seedlings indicated in Fig. 5B, determining the viability (%) of another test (not shown), and calculating the average of two tests.

As is apparent from the test results shown in Fig. 5B and Fig. 6, *Arabidopsis thaliana* cultivated in an allantoin-containing medium has resistance to high temperature stress. Since allantoin is accumulated in plant bodies of *Arabidopsis thaliana* cultivated in an allantoin-containing medium, it was concluded that the high temperature stress resistance observed in this experiment was realized by the presence of allantoin at high concentration in plant bodies.

A phenomenon of chlorosis (whitening) of leaves was observed in plants damaged by high temperature stress.

## Claims

1. A method for improving the high temperature stress resistance of a plant, comprising a step of applying a high temperature stress resistance-improving agent comprising allantoin as an active ingredient to the plant.

2. The method according to claim 1, wherein the step comprises applying the high temperature stress resistance-improving agent to the plant before the plant receives stress.

3. A method for suppressing whitening of a plant due to high temperature stress, comprising a step of applying a whitening suppressor comprising allantoin as an active ingredient to a plant.

4. Use of allantoin for improving the high temperature stress resistance of a plant.

5. The use according to claim 4, for suppressing one or more selected from the group consisting of whitening of the plant due to high temperature stress, withering of the plant due to high temperature stress, and curling of plant leaves due to high temperature stress.

6. The use according to claim 5, for suppressing whitening of a plant due to high temperature stress.

## Patentansprüche

1. Verfahren zur Verbesserung der Hochtemperaturstressbeständigkeit einer Pflanze, umfassend einen Schritt des Aufbringens eines Hochtemperaturstressbeständigkeit verbessernden Mittels umfassend Allantoin als Wirkstoff auf die Pflanze.

2. Verfahren gemäß Anspruch 1, wobei der Schritt umfasst Aufbringen des Hochtemperaturstressbeständigkeit verbessernden Mittels auf die Pflanze, bevor die Pflanze Stress erfährt.

3. Verfahren zur Unterdrückung der Aufhellung einer Pflanze aufgrund von Hochtemperaturstress, umfassend einen Schritt des Aufbringens eines Aufhellungsunterdrückers umfassend Allantoin als Wirkstoff auf eine Pflanze.

4. Verwendung von Allantoin zur Verbesserung der Hochtemperaturstressbeständigkeit einer Pflanze.

5. Verwendung gemäß Anspruch 4, zur Unterdrückung einer oder mehrerer ausgewählt aus der Gruppe, bestehend aus Aufhellung der Pflanze aufgrund von Hochtemperaturstress, Welken der Pflanze aufgrund von Hochtemperaturstress, und Kräuseln der Pflanzenblätter aufgrund von Hochtemperaturstress.

6. Verwendung gemäß Anspruch 5, zur Unterdrückung des Aufhellens einer Pflanze aufgrund von Hochtemperaturstress.

## Revendications

1. Procédé d'amélioration de la résistance au stress des hautes températures d'une plante, comprenant une étape consistant à appliquer à la plante un agent d'amélioration de la résistance au stress des hautes températures comprenant de l'allantoïne comme ingrédient actif.

2. Procédé selon la revendication 1, dans lequel l'étape comprend une application à la plante de l'agent d'amélioration de la résistance au stress des hautes températures avant que la plante ne reçoive un stress.

3. Procédé de suppression du blanchiment d'une plante dû au stress des hautes températures, comprenant une étape consistant à appliquer à une plante un suppresseur de blanchiment comprenant de l'allantoïne comme ingrédient actif.

4. Utilisation d'allantoïne pour améliorer la résistance au stress des hautes températures d'une plante.

5. Utilisation selon la revendication 4, pour supprimer un ou plusieurs sélectionnés dans le groupe consistant en un blanchiment de la plante dû au stress des hautes températures, un flétrissement de la plante dû au stress des hautes températures, et un enroulement de feuilles de plante dû au stress des hautes températures.

6. Utilisation selon la revendication 5, pour supprimer un blanchiment d'une plante dû au stress des hautes températures.
